# EUROPEAN PATENT APPLICATION

(11) **EP 2 453 022 A1**
(43) Date of publication of application: **16.05.2012**
(21) Application number: 10797207.7
(22) Date of filing: 09.07.2010
(51) Int. Cl.: C12Q 1/68, C12M 1/00, C12N 15/09

(54) **METHOD FOR DETECTION OR ANALYSIS OF TARGET SEQUENCE IN GENOMIC DNA**

(30) Priority: 09.07.2009 US 213739 P
(71) Applicant: NGK Insulators, Ltd., Nagoya-shi, Aichi 467-8530 (JP)
(72) Inventor: KAWASE, Mitsuo, Nagoya-shi Aichi 467-8530 (JP); HIROTA, Toshikazu, Nagoya-shi Aichi 467-8530 (JP); NIWA, Kousuke, Nagoya-shi Aichi 467-8530 (JP)
(74) Representative: Paget, Hugh Charles Edward
(86) International application number: PCT/JP2010/061720
(87) International publication number: WO 2011/004896

(57) **Abstract**

An object of the teaching of this specification is to provide a rapid and highly accurate method of testing a target nucleic acid and to provide an apparatus for use in this method.

A method of detecting or analyzing a target sequence in a genomic DNA by using a capture probe immobilized on a solid carrier includes: bringing the target nucleic acid into contact with a first query probe that has a sequence complementary to a portion of the target sequence or to a sequence adjacent to the portion and a second query probe that has a sequence complementary to another portion of the target sequence or to a sequence adjacent to the another portion and that has a recognition sequence complementary to a portion of the capture probe; acquiring a ligated molecule by ligating the first query probe and the second query probe that are hybridized to the target nucleic acid; bringing the ligated molecule into contact with the capture probe on the solid carrier and then capturing the ligated molecule on the solid carrier by hybridizing the capture probe with the recognition sequence in the ligated molecule; and detecting the captured ligated molecule.

### Sequence Listing

SEQUENCE LISTING

<110>  NGK INSULATORS, LTD.

<120>  Method of Detecting or Analyzing Target sequense of DNA

<130>  K09-304-PCT

<160>  109

<170>  PatentIn version 3.3

<210>  1
<211>  23
<212>  DNA
<213>  Artificial

<220>
<223>  probe

<400>  1
tgttctctga ccaatgaatc tgc                                              23

<210>  2
<211>  23
<212>  DNA
<213>  Artificial

<220>
<223>  probe

<400>  2
tggaactggg aacgctttag atg                                              23

<210>  3
<211>  23
<212>  DNA
<213>  Artificial

<220>
<223>  probe

<400>  3
ttcgcttcgt tgtaatttcg gac                                              23

<210>  4
<211>  23
<212>  DNA
<213>  Artificial

<220>
<223>  probe

<400>  4
aggcatccta agaaatcgct act                                              23

<210>  5
<211>  23
<212>  DNA
<213>  Artificial

<220>
<223>  probe

<400>  5
tagcccagtg atttatgaca tgc                                              23


<210>  6
<211>  23
<212>  DNA
<213>  Artificial

<220>
<223>  probe

<400>  6
cgctctggtt actattggac gtt                                              23


<210>  7
<211>  23
<212>  DNA
<213>  Artificial

<220>
<223>  probe

<400>  7
tagccaactc taaataacgg acg                                              23


<210>  8
<211>  23
<212>  DNA
<213>  Artificial

<220>
<223>  probe

<400>  8
ttcggttgtc gatatgagga tct                                              23


<210>  9
<211>  23
<212>  DNA
<213>  Artificial

<220>
<223>  probe

<400>  9
gggggtact tcatacaaga tgc                                               23


<210>  10
<211>  23
<212>  DNA
<213>  Artificial

<220>
<223>  probe

<400>  10
gagtagcagg caaataccct aga                                              23

<210>  11
<211>  23
<212>  DNA
<213>  Artificial

<220>
<223>  probe

<400>  11
gcctattaag gtctacgtca tcg                                              23


<210>  12
<211>  23
<212>  DNA
<213>  Artificial

<220>
<223>  probe

<400>  12
agtcatacag tgaggaccaa atg                                              23


<210>  13
<211>  23
<212>  DNA
<213>  Artificial

<220>
<223>  probe

<400>  13
cattcgacat aagctgttga tgc                                              23


<210>  14
<211>  23
<212>  DNA
<213>  Artificial

<220>
<223>  probe

<400>  14
tgctcactta cattacgtcc atg                                              23


<210>  15
<211>  23
<212>  DNA
<213>  Artificial

<220>
<223>  probe

<400>  15
tacacctatc aactcgtaga gca                                              23


<210>  16
<211>  23
<212>  DNA

<213>  Artificial

<220>
<223>  probe

<400>  16
aggtccggta gtaatttagg tgc                                              23


<210>  17
<211>  23
<212>  DNA
<213>  Artificial

<220>
<223>  probe

<400>  17
tgcactctga tatatacagg cca                                              23


<210>  18
<211>  23
<212>  DNA
<213>  Artificial

<220>
<223>  probe

<400>  18
gcagccctta tagataacgg gac                                              23


<210>  19
<211>  23
<212>  DNA
<213>  Artificial

<220>
<223>  probe

<400>  19
gaagccatga tactgttcag ggt                                              23


<210>  20
<211>  23
<212>  DNA
<213>  Artificial

<220>
<223>  probe

<400>  20
tattctacca acgacatcac tgc                                              23


<210>  21
<211>  23
<212>  DNA
<213>  Artificial

<220>
<223>  probe

<400>  21
ccatcagtta ttcggaggga ctc                                              23


<210>  22
<211>  23
<212>  DNA
<213>  Artificial

<220>
<223>  probe

<400>  22
ccatatccga ttattagcga cgg                                              23


<210>  23
<211>  23
<212>  DNA
<213>  Artificial

<220>
<223>  probe

<400>  23
catctccaag aattgaccca cca                                              23


<210>  24
<211>  23
<212>  DNA
<213>  Artificial

<220>
<223>  probe

<400>  24
ccgtcgtgtt attaaagacc cct                                              23


<210>  25
<211>  23
<212>  DNA
<213>  Artificial

<220>
<223>  probe

<400>  25
gaaggatcgc ttttatctgg cat                                              23


<210>  26
<211>  23
<212>  DNA
<213>  Artificial

<220>
<223>  probe

<400>  26
catttgtcag gtacagtcca ctt                                              23


<210>  27

<211>  23
<212>  DNA
<213>  Artificial

<220>
<223>  probe

<400>  27
gcccacactc ttacttatcg act                                              23


<210>  28
<211>  23
<212>  DNA
<213>  Artificial

<220>
<223>  probe

<400>  28
cgctgttact gtaagcgtac tag                                              23


<210>  29
<211>  23
<212>  DNA
<213>  Artificial

<220>
<223>  probe

<400>  29
cgcgattcct attgattgat ccc                                              23


<210>  30
<211>  23
<212>  DNA
<213>  Artificial

<220>
<223>  probe

<400>  30
ccgtctgggt taaagattgc tag                                              23


<210>  31
<211>  23
<212>  DNA
<213>  Artificial

<220>
<223>  probe

<400>  31
agtcagtcca aatctcagga tgg                                              23


<210>  32
<211>  23
<212>  DNA
<213>  Artificial

<220>

<223>  probe

<400>  32
cgcctaaatg aaactcactc tgc                                               23


<210>  33
<211>  23
<212>  DNA
<213>  Artificial

<220>
<223>  probe

<400>  33
ggggtcaaac caacaattga tct                                               23


<210>  34
<211>  23
<212>  DNA
<213>  Artificial

<220>
<223>  probe

<400>  34
gcccattgat agaattacga ggc                                               23


<210>  35
<211>  23
<212>  DNA
<213>  Artificial

<220>
<223>  probe

<400>  35
atgccgttgt caagagttat ggt                                               23


<210>  36
<211>  23
<212>  DNA
<213>  Artificial

<220>
<223>  probe

<400>  36
tgccggctat cgtaagtata tgc                                               23


<210>  37
<211>  23
<212>  DNA
<213>  Artificial

<220>
<223>  probe

<400>  37
gcacctcata ccttcataga gca                                               23

<210>  38
<211>  23
<212>  DNA
<213>  Artificial

<220>
<223>  probe

<400>  38
cgcgacattt agtccaggag atg                                              23


<210>  39
<211>  23
<212>  DNA
<213>  Artificial

<220>
<223>  probe

<400>  39
ctagtccatt gtaacgaagg cca                                              23


<210>  40
<211>  23
<212>  DNA
<213>  Artificial

<220>
<223>  probe

<400>  40
agacaattag aatcagtgcc cct                                              23


<210>  41
<211>  23
<212>  DNA
<213>  Artificial

<220>
<223>  probe

<400>  41
gcattgaggt attgttgctc cca                                              23


<210>  42
<211>  23
<212>  DNA
<213>  Artificial

<220>
<223>  probe

<400>  42
cgagagtctg taatagccga tgc                                              23


<210>  43
<211>  23
<212>  DNA
<213>  Artificial

<220>
<223>  probe

<400>  43
tgccgtgata cttaactacg cta                                              23


<210>  44
<211>  23
<212>  DNA
<213>  Artificial

<220>
<223>  probe

<400>  44
gagtccgcaa aaatatagga ggc                                              23


<210>  45
<211>  23
<212>  DNA
<213>  Artificial

<220>
<223>  probe

<400>  45
gcctcacata actggagaaa cct                                              23


<210>  46
<211>  23
<212>  DNA
<213>  Artificial

<220>
<223>  probe

<400>  46
cgccaatgac aataagttga ggc                                              23


<210>  47
<211>  23
<212>  DNA
<213>  Artificial

<220>
<223>  probe

<400>  47
cgcgatataa cattaaccga ggc                                              23


<210>  48
<211>  23
<212>  DNA
<213>  Artificial

<220>
<223>  probe

<400>  48

cacgcttagt tcctacctta ggc                                              23

<210>  49
<211>  23
<212>  DNA
<213>  Artificial

<220>
<223>  probe

<400>  49
cgcgtcgaat tacttaatca cca                                              23

<210>  50
<211>  23
<212>  DNA
<213>  Artificial

<220>
<223>  probe

<400>  50
gggataggta ttatgctcca gcc                                              23

<210>  51
<211>  23
<212>  DNA
<213>  Artificial

<220>
<223>  probe

<400>  51
cgccattata caacggttca tgc                                              23

<210>  52
<211>  23
<212>  DNA
<213>  Artificial

<220>
<223>  probe

<400>  52
gcctatatga accaagccac tgc                                              23

<210>  53
<211>  23
<212>  DNA
<213>  Artificial

<220>
<223>  probe

<400>  53
cgccgtcagt acttgtatag atg                                              23

<210>  54
<211>  23

<212>  DNA
<213>  Artificial

<220>
<223>  probe

<400>  54
gtcggtatcg aaaaggtact gca                                              23


<210>  55
<211>  23
<212>  DNA
<213>  Artificial

<220>
<223>  probe

<400>  55
aggcagttca acctatatct gcg                                              23


<210>  56
<211>  23
<212>  DNA
<213>  Artificial

<220>
<223>  probe

<400>  56
ggtcgtaaca ttgagaggag acg                                              23


<210>  57
<211>  23
<212>  DNA
<213>  Artificial

<220>
<223>  probe

<400>  57
ggcgatttat tgctaactgg cta                                              23


<210>  58
<211>  23
<212>  DNA
<213>  Artificial

<220>
<223>  probe

<400>  58
gcactaccgc taactatacg cta                                              23


<210>  59
<211>  23
<212>  DNA
<213>  Artificial

<220>
<223>  probe

<400>  59
ggctcgtagt actccttaca tgc                                              23


<210>  60
<211>  23
<212>  DNA
<213>  Artificial

<220>
<223>  probe

<400>  60
ggctctacaa acttgtgtcc atg                                              23


<210>  61
<211>  23
<212>  DNA
<213>  Artificial

<220>
<223>  probe

<400>  61
ggtggagtga atctcactag act                                              23


<210>  62
<211>  23
<212>  DNA
<213>  Artificial

<220>
<223>  probe

<400>  62
ctagcacaat taatcaatcc gcc                                              23


<210>  63
<211>  23
<212>  DNA
<213>  Artificial

<220>
<223>  probe

<400>  63
gcagctgaat tgctatgatc acc                                              23


<210>  64
<211>  23
<212>  DNA
<213>  Artificial

<220>
<223>  probe

<400>  64
gcctatagtg tcgattgtcc tcg                                              23

<210>  65
<211>  23
<212>  DNA
<213>  Artificial

<220>
<223>  probe

<400>  65
cgatcacgga ttaatgtcac ccc                                              23


<210>  66
<211>  23
<212>  DNA
<213>  Artificial

<220>
<223>  probe

<400>  66
aagagattta acttgagctc gcc                                              23


<210>  67
<211>  23
<212>  DNA
<213>  Artificial

<220>
<223>  probe

<400>  67
tttgttgttc gatatcaggc gtg                                              23


<210>  68
<211>  23
<212>  DNA
<213>  Artificial

<220>
<223>  probe

<400>  68
gcccgggaat agattataac gca                                              23


<210>  69
<211>  23
<212>  DNA
<213>  Artificial

<220>
<223>  probe

<400>  69
gcatttttag taatccgagc gcc                                              23


<210>  70
<211>  23
<212>  DNA
<213>  Artificial

<220>
<223>  probe

<400>  70
catggataag ttttcaagct gcg                                              23


<210>  71
<211>  23
<212>  DNA
<213>  Artificial

<220>
<223>  probe

<400>  71
gagacaggta aaccctcaga gca                                              23


<210>  72
<211>  23
<212>  DNA
<213>  Artificial

<220>
<223>  probe

<400>  72
tagcacccgt taaaacggaa atg                                              23


<210>  73
<211>  23
<212>  DNA
<213>  Artificial

<220>
<223>  probe

<400>  73
tatgtttagt tgttgaaccg gcg                                              23


<210>  74
<211>  23
<212>  DNA
<213>  Artificial

<220>
<223>  probe

<400>  74
cgatcagctc tatttccctc cca                                              23


<210>  75
<211>  23
<212>  DNA
<213>  Artificial

<220>
<223>  probe

<400>  75
agtcagttaa tcagacgtga gca                                              23

<210> 76
<211> 23
<212> DNA
<213> Artificial

<220>
<223> probe

<400> 76
tggcaataca ataacgtatc gcg 23


<210> 77
<211> 23
<212> DNA
<213> Artificial

<220>
<223> probe

<400> 77
cgcagtttgc aagaacgaac aaa 23


<210> 78
<211> 23
<212> DNA
<213> Artificial

<220>
<223> probe

<400> 78
cgcgataatt gatacctacg ggc 23


<210> 79
<211> 23
<212> DNA
<213> Artificial

<220>
<223> probe

<400> 79
ggggtgtgag agctttttag acg 23


<210> 80
<211> 23
<212> DNA
<213> Artificial

<220>
<223> probe

<400> 80
gggatccgta acaagtgtgt tag 23


<210> 81
<211> 23
<212> DNA

<213>  Artificial

<220>
<223>  probe

<400>  81
accactatga ttgaggaaac gcg                                              23


<210>  82
<211>  23
<212>  DNA
<213>  Artificial

<220>
<223>  probe

<400>  82
cgtctttagt atcaaccctc cgc                                              23


<210>  83
<211>  23
<212>  DNA
<213>  Artificial

<220>
<223>  probe

<400>  83
gcatacgaac ttctatatcg gcg                                              23


<210>  84
<211>  23
<212>  DNA
<213>  Artificial

<220>
<223>  probe

<400>  84
ccgtgtgtat gagtatgaca gca                                              23


<210>  85
<211>  23
<212>  DNA
<213>  Artificial

<220>
<223>  probe

<400>  85
tgctgtcttc gtgttttacc tag                                              23


<210>  86
<211>  23
<212>  DNA
<213>  Artificial

<220>
<223>  probe

<400>  86
cgatcatgta aagctaactc gcg                                              23


<210>  87
<211>  23
<212>  DNA
<213>  Artificial

<220>
<223>  probe

<400>  87
tgccgtcatt taaacgtaag ggt                                              23


<210>  88
<211>  23
<212>  DNA
<213>  Artificial

<220>
<223>  probe

<400>  88
tggcaattac agttgttaac gca                                              23


<210>  89
<211>  23
<212>  DNA
<213>  Artificial

<220>
<223>  probe

<400>  89
gagtcgaaga cctcctccta ctc                                              23


<210>  90
<211>  23
<212>  DNA
<213>  Artificial

<220>
<223>  probe

<400>  90
atgccaatat gtactcgtga ctc                                              23


<210>  91
<211>  23
<212>  DNA
<213>  Artificial

<220>
<223>  probe

<400>  91
gcatatagtg acggtaaggc gaa                                              23


<210>  92

<211>  23
<212>  DNA
<213>  Artificial

<220>
<223>  probe

<400>  92
gcctcacttg taataagcgg gac                                              23


<210>  93
<211>  23
<212>  DNA
<213>  Artificial

<220>
<223>  probe

<400>  93
gtcccaaaag cttcttacgg acg                                              23


<210>  94
<211>  23
<212>  DNA
<213>  Artificial

<220>
<223>  probe

<400>  94
ctaggtacaa caccaactgt ctc                                              23


<210>  95
<211>  23
<212>  DNA
<213>  Artificial

<220>
<223>  probe

<400>  95
tgccggttat acctttaagg acg                                              23


<210>  96
<211>  23
<212>  DNA
<213>  Artificial

<220>
<223>  probe

<400>  96
ggctggttaa atgtaaatcc gcg                                              23


<210>  97
<211>  23
<212>  DNA
<213>  Artificial

<220>

<223>  probe

<400>  97
cgcggtacta ttagaaaggg cta                                                23


<210>  98
<211>  23
<212>  DNA
<213>  Artificial

<220>
<223>  probe

<400>  98
agtcgcttaa ttactccgga tgg                                                23


<210>  99
<211>  23
<212>  DNA
<213>  Artificial

<220>
<223>  probe

<400>  99
cgctgttggt attaccttcc tcg                                                23


<210>  100
<211>  23
<212>  DNA
<213>  Artificial

<220>
<223>  probe

<400>  100
tgcagtgtaa gcaactattg tct                                                23


<210>  101
<211>  25
<212>  DNA
<213>  Artificial

<220>
<223>  primer

<400>  101
aggttaaaac tcaaaggaat tgacg                                              25


<210>  102
<211>  21
<212>  DNA
<213>  Artificial

<220>
<223>  primer

<400>  102
atggtgtgac gggcggtgtg t                                                  21

<210>  103
<211>  32
<212>  DNA
<213>  Artificial

<220>
<223>  probe

<400>  103
ccgtgtccac tctagaaaaa ccttgaccac tc                                    32


<210>  104
<211>  32
<212>  DNA
<213>  Artificial

<220>
<223>  probe

<400>  104
ccgtgtccac tctagaaaaa ccttgagcgc aa                                    32


<210>  105
<211>  31
<212>  DNA
<213>  Artificial

<220>
<223>  probe

<400>  105
tagagatagc agattcattg gtcagagaac a                                     31


<210>  106
<211>  31
<212>  DNA
<213>  Artificial

<220>
<223>  probe

<400>  106
tagagataca tctaaagcgt tcccagttcc a                                     31


<210>  107
<211>  23
<212>  DNA
<213>  Artificial

<220>
<223>  primer

<400>  107
ccgtgtccac tctagaaaaa cct                                              23


<210>  108
<211>  15
<212>  DNA
<213>  Artificial

<220>
<223>  probe

<400>  108
accactctag agata                                                        15


<210>  109
<211>  15
<212>  DNA
<213>  Artificial

<220>
<223>  probe

<400>  109
agcgcaatag agata                                                        15

## Description

### Technical Field

The present invention relates to a method for detecting or analyzing a target sequence in a genomic DNA. This application claims priority to United States Provisional Application, US61/213,739, filed on July 9, 2009, the contents of which are hereby incorporated by reference in their entirety into the present application.

### Background Art

The response to an infection of an animal or the contamination of food by a microorganism, for example, a bacterium, requires the reliable and rapid identification of the microorganism. This is due to the speed of microbial proliferation and the necessity for quickly starting eradication of the microorganism and therapeutic maneuvers. Viewed in this context, methods that combine gene amplification technology, e.g., PCR, and hybridization have been proposed as methods for the identification of microorganisms from a sample test material. For example, a DNA microarray has been disclosed for carrying out the detection and identification of a specific bacterium using, as a target nucleic acid, a specific region of the 16S ribosomal RNAgene ofthe bacterium that is the detection target (Patent Document 1). In addition, the detection and identification of oral microbial species related to oral cavity diseases and the use of a DNA microarray for this detection and identification have also been disclosed (Non Patent Document 1). Thus, as shown in FIG. 5, these documents disclose the identification and detection of, e.g., bacteria, by preparing nucleic acid from the bacteria in a sample material and using this nucleic acid as a template; preparing labeled probes against its array probe sequences (target sequences); carrying out hybridization between these labeled probes and oligonucleotides that are based on sequences specific to the species or genus to which the bacterium that is the detection target of these labeled probes belongs; and determining the presence/absence of the occurrence of hybridization.

Detection methods for single nucleotide polymorphism (SNP) typing using DNA microarrays are also known (Non Patent Document 2). As shown in FIG. 6, the following is described in Non Patent Document 2: amplification of only SNP site-containing fragments by multiplex PCR on an extracted genomic DNA; then annealing of the DNA fragments with 5'- and 3'-query probes designed in conformity to the sequences of the PCR products; and subsequent execution of a ligation reaction using a DNA ligase. This ligation product contains a specific sequence, originating with the query probe used, that is complementary to a specific oligonucleotide that is immobilized in a microarray The ligation product, which contains a sequence complementary to the aforementioned SNP site, is labeled by carrying out PCR using two types of fluorescent-labeled primers; this labeled sample is reacted with a microarray in which artificial, mishybridization-free oligonucleotides are immobilized; and, after washing, measurement is carried out using a fluorescent scanner and digitization and analysis are performed.

Hybridization is performed in the methods disclosed in Patent Document 1 and Non Patent Document 1 at a temperature lower than the usual temperatures (37°C to 65°C). Thus, hybridization is performed in the methods of Patent Document 1 and Non Patent Document 1 at a temperature of 37°C to 50°C for about 2 hours. Hybridization is performed for about 30 minutes at 37°C in Non Patent Document 2.

### [Patent Document]

[Patent Document 1] Japanese Patent No. 4,189,002

### [Non Patent Document]

[Non Patent Document 1] Non Patent Document 1: Ministry of Health, Labor and Welfare, Scientific Research Grant, Project for Maintaining and Promoting Consumer Confidence in Food and Safety, Research Related to the Evaluation of the Efficacy of So-Called Health Foods (Fiscal Year 2006, Summary · Collaborator Research Report)
[Non Patent Document 2] Analytical Biochemistry, 364(2007) 78-85; Ono et al., Polym. Prep.Jpn, 55(1), 2090, 2006

### Summary of Invention

Hybridization is run at low temperatures in Patent Document 1 and Non Patent Document 1 in order to increase the quantity of the hybrid formed by hybridization between the labeled probe and the specific-sequence oligonucleotide, but this also has the potential for causing mishybridization. The accuracy of identification may thus also be lowered. In addition, an examination of hybridization at higher temperatures generates the requirement for further examination of the hybridization conditions and probe design for this purpose.

The method disclosed in Non Patent Document 2 is a highly accurate SNP detection method free of mishybridization However, the preparation of other types of probes is required and this is problematic for a rapid and convenient execution in the case of microbial contamination.

An object of the teaching in this Specification is to provide a rapid and highly accurate method of testing for a target nucleic acid and to provide an apparatus for use in this method.

The present inventors sought to improve the method disclosed in Non Patent Document 2 by reducing mishybridization and shortening the hybridization time for detecting a target nucleic acid. It was discovered as a result that the hybridization step could be sped up without using other types of primers by using a DNA fragment (chimeric DNA) acquired by preparing, for a prescribed region (target sequence) in a target nucleic acid, a primer containing a recognition sequence complementary to this probe sequence and a primer containing a tag sequence complementary to a preselected artificial sequence, annealing these primers, and thereafter ligating with a DNA ligase. The teaching of this Specification provides the following.

The teaching of this Specification provides a method of detecting or analyzing a target sequence in a genomic DNA by using a capture probe immobilized on a solid carrier, the method comprising:
bringing the target nucleic acid into contact with a first query probe that has a sequence complementary to a portion of the target sequence or to a sequence adjacent to the portion and a second query probe that has a sequence complementary to another portion of the target sequence or to a sequence adjacent to the another portion and a recognition sequence complementary to a portion of the capture probe;
acquiring a ligated molecule by ligating the first query probe and the second query probe that are hybridized to the target nucleic acid;
bringing the ligated molecule into contact with the capture probe on the solid carrier and than capturing the ligated molecule on the solid carrier by hybridizing the capture probe with the recognition sequence in the ligated molecule; and
detecting the captured ligated molecule.

The contacting step may use a plurality of the first query probes and a plurality of the second query probes in order to bring these probes into simultaneous contact with a plurality of the target nucleic acids. This method may, based on this plurality of target sequences, detect or identify a source organism or source organisms for a single type of genomic DNA or for two or more types of genomic DNAs.

In addition, labeling any selection from the group consisting of the first query probe, the second query probe, and the ligated molecule may be provided prior to the detectiing step, and the detecting step may detect a signal based on this labeling. This labeling step may be a step in which labeling of the ligated molecule is carried out while amplifying the ligated molecule.

The ligated molecule acquisiting step may ligate the first query probe and the second query probe with a ligase. ADNA amplificating step may be provided prior to the contact step.

The genomic DNA may originate from any selection from viruses and microorganisms that are targets for diagnosis or testing in case of food sanitation and diseases, and the detection step may detect or identify the viruses or microorganisms.

The teaching of this Specification also provides a microarray that is used in the above-described method, the microarray being **characterized in that** the aforementioned capture probe immobilized in the microarray is composed of at least one of the aforementioned capture probes having any base sequence selected from the base sequences described in SEQ ID NOs: 1 to 100 and base sequences that are complementary to these base sequences.

### Brief Description of Drawings

FIG. 1 shows an outline of the detection method disclosed in this Specification.
FIG. 2 shows an outline of a solid article and an example of the capture probe that are used in the detection method disclosed in this Specification.
FIG. 3 shows the results of the detection of a hybridization product obtained in the embodiment.
FIG. 4 shows the results of the detection of a hybridization product obtained in the comparative example.

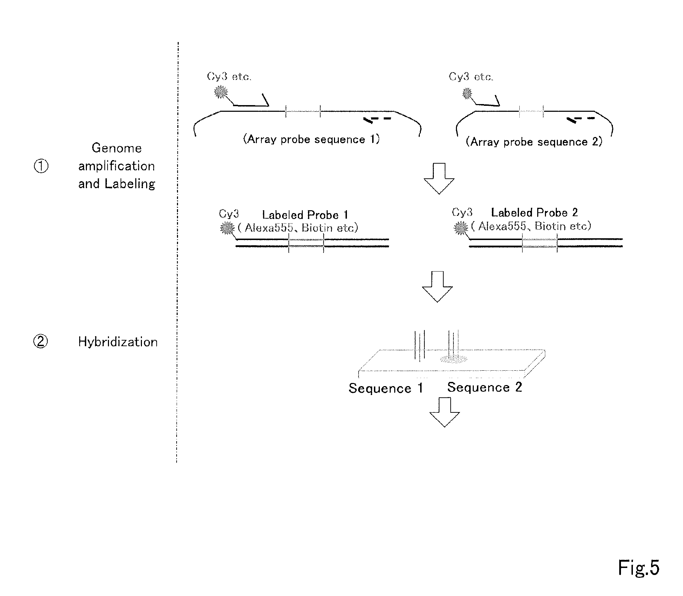

FIG. 5 shows an outline of the detection methods disclosed in Patent Document 1 and Non Patent Document 1.

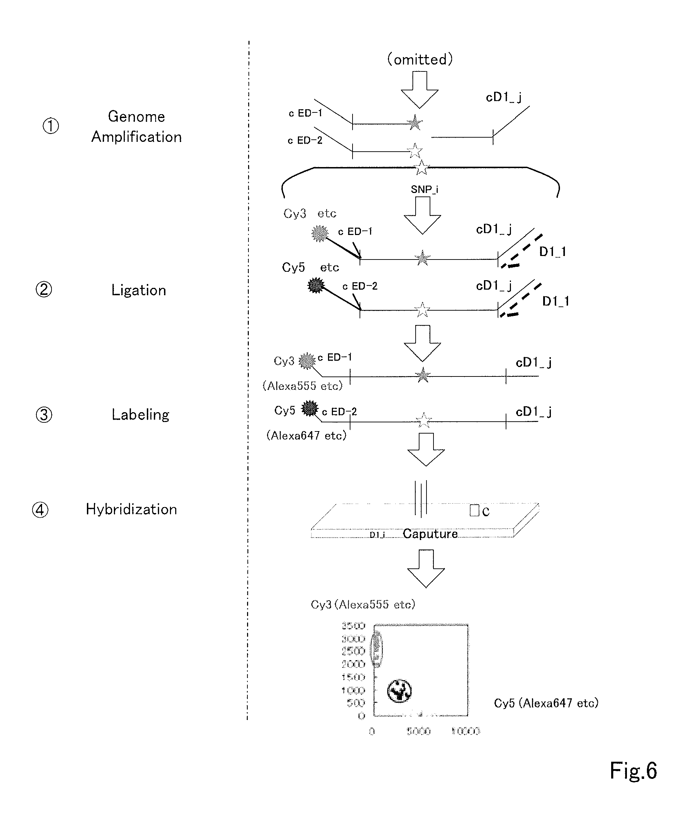

FIG. 6 shows an outline of the detection method disclosed in Non Patent Document 2.

### Description of Embodiments

The teaching in this Specification relates to a method of detecting or analyzing a target sequence in a genomic DNA using a capture probe immobilized on a solid carrier and further relates to an array for this method. In accordance with the teaching in this Specification, the time required in the hybridization step for detection of a nucleic acid in a particular genome and for sequence determination can be shortened and the nonspecific binding to the DNA microarray of a labeled nucleic acid in a particular genome can be reduced.

In the description that follows, a recognition sequence present in the second query probe and complementary to a portion of a capture probe is indicated by recognition sequence (-), while the sequence of the portion present in the capture probe (and complementary to the recognition sequence (-)) is indicated by recognition sequence (+).

According to the method disclosed in this Specification, the detection of the target sequence is performed substantially in the contact step and ligation step with the first query probe and second query probe for the target sequence. Thus, mishybridization in hybridization can be facilitated and its accuracy can be raised by carrying out hybridization between the capture probe and a ligated molecule obtained by ligation after contact by the first and second query probes with the target nucleic acid. According to this method, the capture probe used in hybridization is preferably not intrinsic to the target sequence. Accordingly, a recognition sequence specific to the capture probe used can, when mishybridization is effectively reduced under the prescribed conditions, effectively reduce mishybridization. Similarly, hybridization can be sped up when optimization for low temperature hybridization is performed.

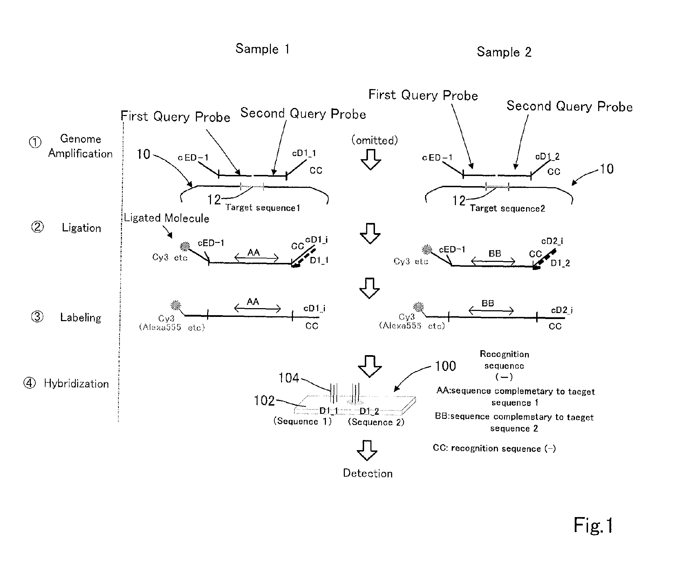

An outline of the teaching of this Specification is shown in FIG. 1. FIG. 1 is an example of the teaching of this Specification and does not limit the teaching of this Specification. As shown in FIG. 1, among the nucleic acid sequences in the extracted DNA, nucleic acid amplification (approximately 100 bp to 1 kbp) is carried out using a polymerase such that the probe sequence region is incorporated. Probes for ligation are then prepared by designing 5'- and 3'-query probes (the first query probe and the second query probe) for the target sequence region in the nucleic acid in the sample test material, based on a consideration of the target sequence in the sample test material and the pertinent recognition sequence in the capture probes, which are preliminarily prepared on a solid carrier and have respective prescribed recognition sequences. These query probes are then annealed on the PCR amplification product and a ligated molecule is subsequently acquired by ligation using a DNA ligase. PCR is carried out using primers labeled with, for example, a fluorescent dye, e.g., Cy, Alexa, and so forth, or with biotin in order to label the ligated molecule. The labeled ligated molecule is brought into contact with the capture probe on the solid carrier and hybridization is detected.

In this Specification, "nucleic acid" encompasses all DNAs and RNAs including cDNA, genomic DNA, synthetic DNA, mRNA, total RNA, hnRNA, and synthetic RNA as well as artificial synthetic nucleic acids such as peptide nucleic acids, morpholino nucleic acids, methylphosphonate nucleic acids, S-oligo nucleic acids, and so forth, The nucleic acid may be single stranded or double stranded. In this Specification, the "target nucleic acid" is any nucleic acid having any sequence. Atypical example is a nucleic acid that can have a base sequence that forms a marker on a gene in a human or in a nonhuman animal for disease onset, disease diagnosis, treatment prognosis, drug selection, treatment selection, and so forth, with regard to health status, a genetic disease, a specific disease such as cancer, and so forth. This marker can be exemplified by a polymorphism, such as an SNP, or a congenital or acquired mutation. The target nucleic acid also encompasses nucleic acid of microbial origin, for example, from a pathogenic bacteria or a virus.

The sample test material, infra, or a nucleic acid fraction therefrom, may also be directly used as the target nucleic acid; however, the use is preferred of an amplification product provided by the amplification - preferably by a PCR amplification reaction and more preferably by a multiplex PCR amplification reaction - of all of a plurality of target nucleic acids,

In this Specification, "target sequence" refers to a characteristic sequence comprising one base or two or more bases in the target nucleic acid of the detection target. For example, it may be a partial sequence of low homology among target nucleic acids or may be a sequence having a low complementarity or homology with other nucleic acids that may be present in the sample. The target sequence may be a sequence characteristic of a target nucleic acid. In addition, it may be a sequence specific to a genus or species of an organism, for example, a microorganism. This target sequence may also be a target sequence with an artificially modified sequence.

The "sample test material" in this Specification is a sample that may contain a target nucleic acid. Any sample that contains a nucleic acid can be used as the sample, including cells, tissue, blood, urine, saliva, and so forth. A nucleic acid-containing fraction can be recovered from such samples by the individual skilled in the art with reference to the pertinent conventional art.

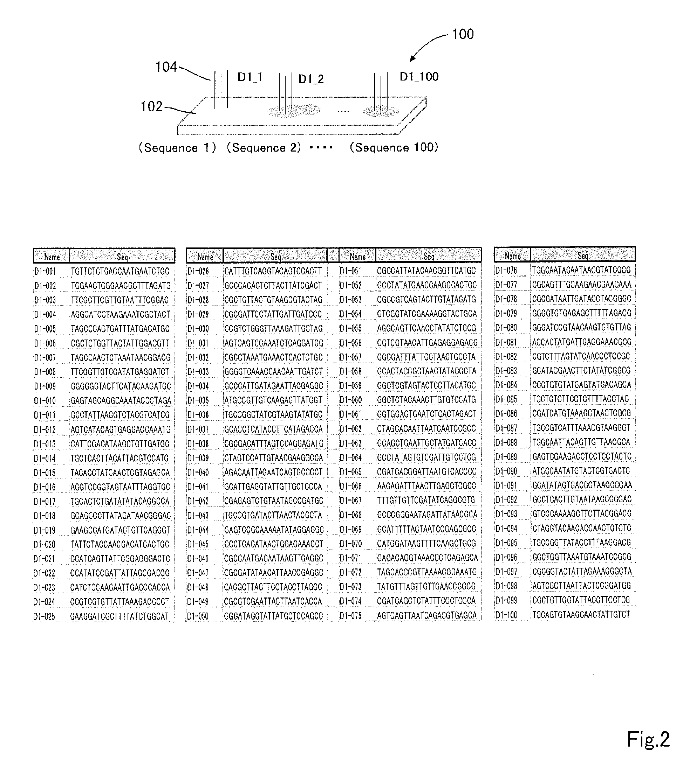

Embodiments of the present invention are described in detail herebelow with reference to the pertinent figures. FIG. 2 shows a solid article that may be used in the detection or analysis method disclosed in this Specification and also shows recognition sequences that may be incorporated in the capture probes and used in the detection or analysis method disclosed in this Specification.

### (Method for detecting or analyzing a target sequence in a genomic DNA)

The method disclosed in this Specification for detecting or analyzing a target sequence may comprise: a step of bringing the aforementioned target nucleic acid into contact with a first query probe that has a sequence complementary to a portion of the target sequence or to a sequence adjacent to the portion and a second query probe that has a sequence complementary to another portion of the target sequence or to a sequence adjacent to the another portion and that has a recognition sequence (-) complementary to a recognition sequence (+) held by the aforementioned capture probe (the contact step); a step of acquiring a ligated molecule by ligating the first query probe and the second query probe that are hybridized to the target nucleic acid (the ligation step); a step of bringing the ligated molecule into contact with the capture probe on the solid carrier and then capturing the ligated molecule on the aforementioned solid carrier by hybridizing the capture probe with the recognition sequence (-) in the ligated molecule (the hybridization step); and a step of detecting the captured ligated molecule (the detection step).

The detection and analysis method disclosed in this Specification (referred to hereafter simply as the present method) uses a solid article on which a capture probe containing a specified recognition sequence (+) is immobilized. Accordingly, the preparation of the solid carrier is described first below followed by a description of each step in the present method.

### (Preparation of the solid carrier)

The solid article 100 that is used for display by the present method may be prepared in advance prior to the execution of the present method, or may be commercially acquired, or may be prepared at each execution of the detection method.

The solid article 100 may comprise a solid carrier 102 provided thereon with a plurality of capture probes 104 that each contain a recognition sequence (+) that is a specific base sequence that differs thereamong. An examination of probe design and synthesis, array fabrication, and hybridization conditions can be circumvented by the preparation of such a solid article 100.

The capture probe 104 has a recognition sequence (+) that is a base sequence specific for each probing. This recognition sequence (+) can be designed independently from the characteristic sequence in the target nucleic acid 10, i.e., the target sequence 12. By carrying out this design independently from the target sequence 12, the recognition sequence (+) in a capture probe 104 can be designed so as to inhibit or avoid nonspecific binding among the plurality of capture probes 104 and can be designed taking into account optimal hybridization conditions, e.g., temperature and time, for the hybridization. Moreover, the same capture probe 104 can always be used regardless of the type of the target nucleic acid 10.

The recognition sequence (+) for a capture probe 104 can, for example, be suitably selected from the base sequences shown in FIG. 2 and Table 1 (SEQ ID NOs: 1 to 100) and their complementary base sequences. These base sequences all have the same base length and have a melting temperature (Tm) of from at least 40°C to not more than 80°C and preferably from at least 50°C to not more than 70°C and can provide uniform hybridization results for hybridization under the same conditions.

A suitable selection from such candidate base sequences can be used for the recognition sequence (+) of a capture probe 104. The melting temperatures for two or more types of capture probes 104 used are preferably as close as possible.

The melting temperature used can be calculated by, for example, the GC% method, Wallace method, and procedures in accordance with Current Protocols in Molecular Biology (described on page 25, Bio Experiments Illustrated 3, "Real Amplification by PCR", Shujunsha Co., Ltd.), but calculation by the nearest neighbor method - which can incorporate the influence of the base sequence concentration and melting temperature range - is preferred in the present invention. The melting temperature by the nearest neighbor method can be readily obtained using Visual OMP software (Tomy Digital Biology Co., Ltd.) or software (OligoCalculator, http://www.ngrl.co.jp/too/ngrl_too1.html) provided by Nihon Gene Research Laboratories Inc. (http://www.ngrl.co.jp/).

The recognition sequence in this capture probe 104 could be an orthonormalized sequence and may be designed, for example, by carrying out calculation of the continuous match length versus DNA sequences of prescribed base length obtained from random numbers and calculation ofthe predicted melting temperature by the nearest neighbor method, the Hamming distance, and predicted secondary structures. Orthonormalized base sequences refer to base sequences that are nucleic acid base sequences that have a uniform melting temperature, i.e., are sequences designed to have melting temperatures that lie within a prescribed range; that do not inhibit hybrid formation with a complementary sequence through formation of an intramolecular structure by the nucleic acid itself; and that do not form stable hybrids except with base sequences complementary thereto. A sequence that is a member of a group of orthonormalized sequences resists reactions between sequences, except for a desired combination, and resists reactions within the sequence itself or else does not engage in these reactions at all. In addition, an orthonormalized sequence, when amplified by PCR, has the property whereby nucleic acid in the amount corresponding to the initial amount of the nucleic acid having an orthonormalized sequence, undergoes quantitative amplification. The details of these orthonormalized sequences are described in H. Yoshida and A. Suyama, "Solution to 3-SAT by breadth first search", DIMACS, Volume 54, 9-20 (2000) and Japanese Patent Application No. 2003-108126. Orthonormalized sequences can be designed using the methods described in these documents.

The capture probe 104 is immobilized on a carrier 102. A solid carrier is preferably used for this carrier 102. For example, the carrier 102 may be plastic or glass, but the material thereof is not particularly limited. The shape of the carrier 102 may be a flat plate shape as shown in FIG. 1 or may be a bead shape, but the shape is not particularly limited. The solid article 100 is preferably an array (particularly a microarray) wherein the carrier 102 is a flat, solid plate and a plurality of capture probes 104 is immobilized in a prescribed arrangement. The array is advantageously set up by immobilizing multiple capture probes 104 in order to comprehensively and simultaneously detect various types of target nucleic acids 10. In addition, the solid article 100 may be provided with a plurality of partitioned array regions on the cartier 102. Sets of capture probes 104 each comprising the same combination may be immobilized in this plurality of array regions, or sets of capture probes 104 each comprising a distinct combination may be immobilized in the plurality of array regions. By immobilizing sets of different combinations of the capture probes 104 in the plurality of array regions, the individual array regions can be assigned to the detection of target nucleic acids 10 in different genes.

A preferred solid article 100 may be provided with an arrangement configuration in which two or more types of the capture probes 104 are arranged in a sequence that is based on their melting temperatures. For example, by using such a solid article 100 and assigning - along the sequence of the capture probes 104 - two or more types of capture probes 104 that correspond to two or more types of target nucleic acids 10 that in turn correspond to two or more types of target sequences 12 that may be present relative to a certain site in a certain gene, the hybridization scatter originating in the immobilization positions of the capture probes 104 and differences in the melting temperatures of the recognition sequences of the capture probes 104, can be inhibited and the target nucleic acid(s) 10 in a sample can be accurately detected

The mode of immobilization of the capture probe 104 is not particularly limited. Covalent bonding or noncovalent bonding may be used. The capture probe 104 may be immobilized on the surface ofthe carrier 102 by various heretofore known methods. In addition, a suitable linker sequence may be provided to the surface of the carrier 102. The linker sequence preferably has the same base length and the same sequence among the capture probes 104.

### (The target nucleic acid amplification step)

The present method may be provided, prior to the contact step, with a step of amplifying the target nucleic acid in the sample test material. The implementation of an amplification step can speed up and increase the efficiency of the contact step and the ligation step. The amplification of a single target nucleic acid in the sample test material or two or more target nucleic acids in the sample test material can be carried out in the amplification step. Thus, the amplification step can be carried out, for example, by PCR, by suitably preparing primer sets in conformity to the types (number) of target nucleic acids to be amplified and/or detected.

### (The contact step)

The contact step is a step of bringing the target nucleic acid into contact with a first query probe, which has a sequence complementary to a portion of a target sequence in a target nucleic acid or to a sequence adjacent to the portion, and a second query probe, which is provided with a sequence complementary to another portion of the aforementioned target sequence or to a sequence adjacent to the another portion and with a recognition sequence (-) complementary to a portion of the aforementioned capture probe. When a target nucleic acid is present in the sample test material, a target sequence in the target nucleic acid can be detected in the contact step by the first query probe and the second query probe. A first query probe and a second query probe are prepared for each target nucleic acid, i.e., for each target sequence. The contact step may also be a step in which a plurality of first query probes and a plurality of second query probes are used and a plurality of target nucleic acids are simultaneously brought into contact with these.

As shown in FIG. 1, the first query probe contains a sequence complementary to a target sequence. For the target sequence, a sequence is selected that can detect the target nucleic acid and can detect and identify the detection target. The length of the target sequence is not particularly limited, but a length of from several bases to about 10 bases can be used. The first query probe may contain a sequence complementary to all or a portion of this target sequence. It is preferably designed so as to contain a sequence complementary to the entire target sequence. The first query probe may contain a sequence complementary to a sequence adjacent to the target sequence in a range at which the specificity of hybridization can be ensured. The sequence complementary to the target sequence is preferably disposed so as to constitute the 3' terminal ofthe first query probe.

A sequence used to provide a label in a later stage may be provided at the 5' terminal side of the first query probe. For example, a prescribed sequence may be disposed in conformity to the type of dye.

The second query probe has a sequence complementary to another portion of the target sequence or to a sequence adjacent to this another portion. As shown in FIG. 1, when the first query probe is provided with a sequence complementary to the entire target sequence, the second query probe preferably has a sequence complementary to an adjacent sequence contiguous with the target sequence. In addition, when the first query probe is provided with a complementary sequence only for a portion of the target sequence, the second query probe then has a sequence complementary to the remaining sequence of the target sequence. This complementary sequence is positioned at the 5' terminal of the second query probe. This 5' terminal is preferably phosphorylated due to the ensuing DNA ligase-mediated ligation step.

The second query probe is provided with a recognition sequence (-) that is complementary to a portion (the recognition sequence (+)) of a capture probe 104. This recognition sequence (-) is correlated in advance with the recognition sequence (+) of a particular capture probe 104 immobilized on the solid carrier 102. The recognition sequence (-) is disposed at the 3' terminal of the second query probe.

The conditions in the contact step are not particularly limited. An ordinary hybridization medium can be used. In order to anneal the first query probe and the second query probe to the target sequence, the target nucleic acid (which is ordinarily double-stranded) recovered from the sample test material must be heated and melted. Thus, the contact step may be accompanied by sufficient heating to achieve this melting. The annealing of these query probes to the target sequence is subsequently realized by lowering the temperature to a suitable temperature. In addition, the contact step need not be executed as an independent step and may be a part of the ligation step. Thus, the ligation step may be carried out at the same time as the contact step.

### (The ligation step)

The ligation step is a step that provides a ligated molecule by ligating the first query probe and second query probe that have hybridized to a target sequence of a target nucleic acid. In this step, the first query probe and second query probe are ligated to each other while they reside in a state of hybridization to the target sequence. Generally, the 3' terminal of the first query probe is ligated with the 5' terminal of the second query probe through the action of a DNA ligase. The ligation step can be carried out in a state in which the annealed state by the query probes to the target sequence is maintained and under conditions in which the ligase used is functional. Thus, the contact step and ligation step can be executed as a single step by considering the annealing temperature and the ligation reaction temperature. When a heat-resistant ligase is used, these can easily be run as a single step.

### (The labeling step)

The present method may be provided, prior to the detection step described below, with a step of labeling any selection from the group consisting of the first query probe, the second query probe, and the ligated molecule. The labeling of any of these provides the ligated molecule with a label and makes possible the detection of hybridization based on the detection in the detection step of labeling at a specific capture probe.

The label may be attached in advance to the first query probe or the second query probe, but the attachment of the label to the ligated molecule provides for a more efficient use of the label. A PCR-based amplification step can be used for attachment of the label. For example, as shown in FIG. 1, an asymmetric PCR may be run using a primer that has a label at the 5' terminal and that contains the same sequence as the sequence at the 5' terminal side of the first query probe and using a probe that has a recognition sequence (-) complementary to the recognition sequence (+) of the second query probe. Such a labeling step is efficient for carrying out labeling of the ligated molecule while amplifying the ligated molecule.

An appropriate selection from the heretofore known labels can be used as the label. The label may be any of various dyes, e.g., fluorescent substances that emit a fluorescent signal when the substance itself is excited, or may be a substance that, in combination with a second component, emits any of various signals by an enzymatic reaction or an antigen-antibody reaction Fluorescent labels such as Cy3, Alexa555, Cy5, and Alexa647 are typically used. In addition, detection based on color generation, for example, by treatment with a substrate using the biotin/streptavidin HPR combination, may also be used.

### (The hybridization step)

The hybridization step is a step of bringing the ligated molecule into contact with the capture probe 104 on the solid carrier and capturing the ligated molecule on the solid carrier by hybridization between the recognition sequence (+) in the capture probe 104 and the recognition sequence (-) in the ligated molecule. This step results in specific hybridization by the ligated molecule to a particular capture probe 104 based on pairing between the recognition sequences (+) (-), with the formation of a double strand. This makes possible as a result the detection of a target sequence that has been correlated in advance to a specific capture probe 104.

According to the present method, only in those instances in which a target nucleic acid is present in the sample test material does the synthesis occur of a ligated molecule having a recognition sequence (-) for a capture probe 104 that has been correlated in advance to the target nucleic acid, while a ligated molecule is not synthesized when the target nucleic acid is not present. In addition, the capture probe 104 is an artificial sequence for which mishybridization is strongly inhibited. Accordingly, the generation of mishybridization between a capture probe 104 and a ligated molecule is strongly inhibited in the hybridization step of the present method. A suitable washing step may also be incorporated after the hybridization step.

### (The detection step)

The detection step is a step of detecting a ligated molecule that has been captured by a capture probe 104. There are no particular limitations on the method of detecting the hybridization product in the detection step. When the ligated molecule has a label, this label may be detected. In addition, double strand detection may be carried out by, for example, an electrical detection method.

The presence/absence and proportion of a target nucleic acid in a test sample can be detected by the detection in the detection step of, for example, a label. According to the present method, the target sequences that are the detection targets can be reliably detected even when detection is simultaneously performed on a plurality of target nucleic acids.

Known detection methodologies, e.g., chemical detection methodologies, electrical detection methodologies, and so forth, can be used as appropriate to detect the ligated molecule captured on the capture probe 104. The presence/absence and proportion of a target nucleic acid 10 can be elucidated from the signal strength information obtained by these detection methodologies.

According to the present method, a target sequence-containing DNA fragment (the ligated molecule) is acquired using a first query probe, which has a sequence complementary to a target sequence specific to a target nucleic acid that originates from a detection target, and using a second query probe, which contains a sequence complementary to a portion of this target sequence or to a sequence adjacent to this portion and which also contains a recognition sequence (-) that has been correlated in advance with a highly selective recognition sequence (+). This ligated molecule is then brought into contact with a capture probe that contains the recognition sequence (+) to form a hybridization product, which is detected. A ligated molecule is acquired selectively with respect to a target sequence in the contact step and ligation step in the present method, and the hybridization step just has to check for, with respect to the ligated molecule, the efficient and highly selective formation of a hybrid between the recognition sequences (+)(-). As a result, the present method can improve the accuracy of and time requirement for the hybridization step, which has heretofore been a bottleneck. In addition, the time and labor required for an examination of conditions can be eliminated in relation to the hybridization step.

As described above, starting from a nucleic acid, e.g., a DNA that is a test target, the source organism for this DNA can be identified using the present method. Accordingly, the present method is useful as a test method for checking for the presence/absence of various organisms that may be test targets. In particular, the present method is useful as a test method for, e.g., viruses and microorganisms that are test targets for food hygiene and safety and targets for disease diagnosis and testing.

### (The probe set)

The teaching ofthis Specification provides a probe set comprising the previously described first query probe and second query probe. This probe set is used in combination with a solid article 100 on which a capture probe 104 is immobilized and is a probe set adapted for acquiring the previously described ligated molecule. The first query probe, for example, has a sequence complementary to a target sequence that enables the detection, relative to a single gene, of a mutation present in an individual or differences present in a species or genus and has a recognition sequence (-) correlated to a capture probe 104, while the second query probe has a sequence complementary to another portion of the target sequence or to a sequence adjacent to the another portion and has a recognition sequence (-) complementary to the recognition sequence (+) present in a preliminarily correlated capture probe. The probe set may comprise at least one such first query probe and second query probe or may comprise two or more types of probe sets in order to detect two or more types of target nucleic acids.

The present invention is specifically described below using an embodiment, but the present invention is not limited to the following embodiment.

### First Embodiment

A target sequence in a specified genome was detected and identified in this embodiment.

### (1) Fabrication of the DNA microarray

Using for the capture probe an aqueous solution prepared by dissolving a synthetic oligo DNA (Nihon Gene Research Laboratories Inc.) modified by the amino group at the 3' terminal, a geneslide (Toyo Kohan Co., Ltd.) glass slide was spotted using a GEIVESHOT (registered trademark) spotter from NGK Insulators, Ltd. The synthetic oligo DNA sequences used were the 100 species D1_1 to D1_100 described in Supplementary Table 1 in the literature, Analytical Biochemistry, 364(2007) 78-85. (Table 1).

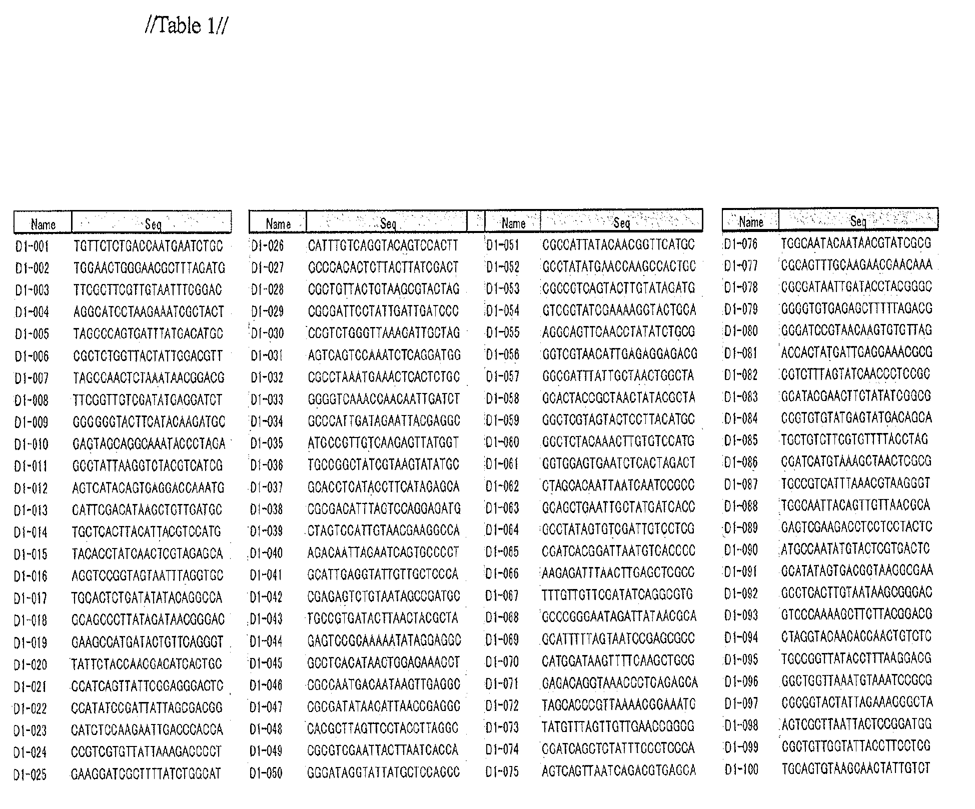

After each of these synthetic oligonucleotides had been spotted on a slide, baking was carried out 15 for 1 hour at 80°C. The synthetic oligo DNA was then immobilized and washed using the following procedure. After baking, washing was carried out for 15 minutes with 2× SSC/0.2% SDS followed by washing for 5 minutes at 95°C with 2x SSC/0.2% SDS. Washing was then carried out a total of 3 times with sterile water (shaking up and down 10 times), and liquid elimination was performed by centrifugation (1000 rpm × 3 minutes).

### (2) Amplification of the target nucleic acid

Among the microbial species present in the oral cavity, *Enterococcus faecalis* and *Pseudoramibacter alactolyticus* were used as the detection target microorganisms, The following primers were used in order to amplify target nucleic acids originating with each of these microorganisms. These primers are designed to amplify a specific portion of the 16S rRNAgene region of these microorganisms.
F-primer: 5'-AGGTTAAAACTCAAAGGAATTGACG-3'(SEQ ID NO: 101)
R-primer: 5'-ATGGTGTGACGCGCGGTGTGT-3' (SEQ ID NO: 102)

PCR was run under the conditions given below on the DNA extracted from these microorganisms to obtain the following amplification products: sample 1 originating from *Enterococcus faecalis* and sample 2 originating from *Pseudoramibacter alactolyticus.* These samples were purified using a MinElute PCR Purification Kit from QIAGEN followed by confirmation that amplification had occurred for the intended length.

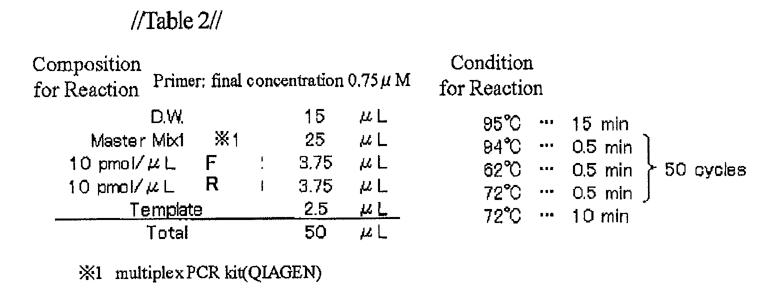

### (3) Annealing to the amplification product and the ligation reaction

The query probes were then annealed to samples 1 and 2, which were the amplification products, and a ligation reaction was carried out. Taq DNA Ligase (Catalog #M0208S) from New England Biolabs was used for the ligation reaction. A GeneAmp PCR System 9700 from Applied Biosystems was used as the thermal cycler for heating. The first query probe and second query probe that were used are shown below. The 5'-phosphorylated second query probe was used in each case. Query 1-1 and query 2-1 are a probe set for amplifying the target nucleic acid in sample 1, while query 2-1 and query 2-2 are a probe set for amplifying the target nucleic acid in sample 2. The italicized letters in the first and second query probes refer to the sequence complementary to the target sequence. The underlining in the second query probe is a recognition sequence (-) for each of the preliminarily correlated capture probes.

**∥Table 3∥**

| Probe Name | sequence(5' → 3') |
|---|---|
| Query 1 - 1 | CCGTGTCCACTCTAGAAAAACCT*TGACCACTC*(SEQ ID:103) |
| Query 1 - 2 | CCGTGTCCACTCTAGAAAAACCT*TGAGCGCAA* (SEQ ID: 04) |
| | |

| Second Query Probe | |
|---|---|
| Probe Name | sequence(5'→ 3') |
| Query 2 - 1 | *TAGATAGCA*GATTCATTGGTCAGAGAACA (SEQ ID:105) |
| Query 2 - 2 | *TAGAGATA*CATCTAAAGCGTTCCCAGTTCCA (SEQ ID:106) |

Annealing and ligation were carried out as follows. After preparation of the reagent with the composition given below, heating was performed for 5 minutes at 95°C, 1 minute at 50°C, and then 60 minutes at 58°C, followed by a reduction to 10°C. Of the 15 samples obtained, a ligated molecule was made for each sample. The following three combinations were used for the amplification product sample: 3 µL sample + 3 µL sterile water, 3 µL sample 2 + 3 µL sterile water, and 3 µL sample 1 + 3 µL sample 2.

### (Reagent)

| | |
|---|---|
| first query probe/second query probe mix (2.5 µM, each) | 0.300 µL |
| Taq DNA Ligase buffer (10×) | 1.500 µL |
| dH₂O | 1.075 µL |
| Taq DNAligase (40 Units/ µL) | 0.125 µL |
| Subtotal | 3.000 µL |
| PCR product (*) | 6.000 µL |
| total | 9.000 µL |

### (4) Fluorescent labeling of the ligated molecule

Fluorescent labeling of the ligated molecule was performed by carrying out PCR using a labeled primer. TaKaRa Ex Taq® (Catalog# RROOIB) from Takara Bio Inc. was used for the labeling. The GeneAmp PCR System 9700 from Applied Biosystems was used for the thermal cycler. Here, the labeling reagent for the labeled primer can be matched to the specifications of the scanner, e.g., Cy3, 5, Alexa555, 647, and so forth.

The labeled primer and unlabeled primers shown in the table below were first prepared. The labeled primer has the same sequence as the 5' terminal side of the first query probe of the ligated molecule, while the unlabeled primer has a sequence complementary to the recognition sequence (-) of the second query probe of the ligated molecule.

**//Table 4//**

| Labeled ED-[Primer(5'end Alexa555 or 647 Labeled) | |
|---|---|
| Primer Name | Sequence(5' → 3') |
| Alexa-ED-1 | CCGTGTCCACTCTAGAAAAACCT (SEQ ID:107) |
| | |

| D1 Primer | |
|---|---|
| Primer Name | Sequence(5'→ 3') |
| D1_001 | TGTTCTCTGACCAATGAATCTGC |
| D1_002 | TGGAACTGGGAACGCTTTAGATG |

The labeling reaction was run by preparing the reagent shown below and then performing a thermal cycler reaction (1 minute/95°C, then 30 cycles of 30 seconds/95°C → 6 minutes/55°C → 30 seconds/72°C, then lowering to 10°C) using this reagent.

### (Reagent)

| | |
|---|---|
| D1 primer mix (50 µM, each) | 0.12 µL |
| Alexa555-ED-1 (200 µM) | 0.24 µL |
| Ex Taq buffer (10x) | 1.20 µL |
| dNTP mix (2.5 nM) | 0.96 µL |
| Ex Taq polymerase (5 Unit/µL) | 0.12 µL |
| MiliQ | 8.36 µL |
| Subtotal | 11.00 µL |
| ligated molecule | 1.00 µL |
| total | 12.00 µL |

### (5) Hybridization using the DNA microarray

The reaction with the DNA microarray using the labeled samples and the detection thereof were carried out using the following procedures. A labeled sample solution with the composition given below was first prepared and the prepared labeled sample solution was heated for 1 minute at 90°C using a GeneAmp PCR System 9700 from Applied Biosystems and was then heated for 1 minute at 80°C using a heating block (DTLT N from TAITEC). After the treatment, 9 µL of each labeled sample solution was applied to the spot area of a DNA microarray, and, using a Thermoblock Slide (eppendorf) for the comfort/plus to prevent drying, a hybridization reaction was nm by standing for 60 minutes at 37°C.

### (Preparation of the labeled sample solution for hybridization)

| | |
|---|---|
| Hybri Control* | 1.5 µL |
| Hybri Solution* | 9.0 µL |
| Subtotal | 10.5 µL |
| Labeled products | 7.5 µL |
| total | 18.0 µL |
| Alexa555-rD1_100(100nM) | 10 µL |
| TE(pH8.0) | 390 µL |
| total | 400 µL |
| *Hybri Solution composition (2.5 nM) | |
| 20×SSC | 2.0 mL |
| 10% SDS | 0.8 mL |
| 100% formamide | 12.0 mL |
| 100 mM EDTA | 0.8 mL |
| MilliQ | 24.4 mL |
| total | 40.0 mL |

| | |
|---|---|
| *Hybri Control composition (2.5 nM, each) *The Alexa555-labeled oligo DNA sequence used in the Hybri Control was provided by the Alexa555 labeling of the 5' terminal of a sequence complementary to the D1_100 sequence among the probes for the DNA microarray, | |

A wash solution with the composition given below was then prepared and washing was carried out. Thus, the wash solution was transferred to a glass staining tank; the glass slide was immersed after the completion of the hybridization reaction; and up-and-down shaking was performed for 5 minutes. The glass slide was then transferred to a glass staining tank containing sterile water and up-and-down shaking was performed for 1 minute. Centrifugal drying was thereafter carried out for 1 minute at 2000 rpm to remove the moisture remaining on the surface ofthe glass slide.

### (Preparation ofthe wash solution)

| | |
|---|---|
| MilliQ | 188.0 mL |
| 20×SSC | 10.0 mL |
| 10% SDS | 2.0 mL |
| total | 200.0 mL |

### (6) Detection of fluorescence with a scanner

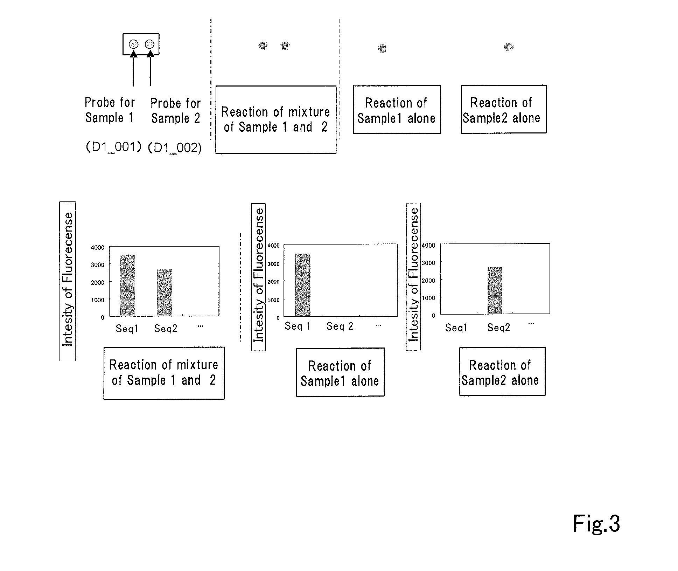

Using an ArrayWoRx from Applied Precision, a fluorescent image was acquired with adjustment of the photoexposure time as appropriate. Digitization was carried out on the fluorescent signal in the obtained image using GenePix Pro. The results are shown in FIG 3.

### (7) Data analysis

As shown in FIG 3, when the reaction was run using a mixture of sample 1 and sample 2, a fluorescent signal was yielded by the reaction for both samples, which indicated that sample detection was possible. When individual reactions were run without mixing the samples, a fluorescent signal was not obtained (not more than 1%) for the nontarget probe, in contrast to the results for conventional methods, and it was thus found that nonspecific reactions by the sample can be substantially reduced (the detection performance is improved).

In the preceding embodiment, nonspecific hybridization was inhibited in the hybridization step even though this step was 60 minutes, and it was thus demonstrated that the present method exhibits an excellent accuracy and speed. When the capture probes shown in Table 1 were used, it was found that the hybridization step could be carried out even in about 30 minutes, and when run for 30 minutes for confirmation, the same results as at 60 minutes could be obtained.

In addition, conventional methods have also required an optimization of the hybridization conditions until desirable results are obtained as well as redesign of the probe sequences for the array and re-conshuction of the array, but in contrast to this the present invention does not require the redesign of the probe sequences for the array and does not require re-construction of the array and makes it possible to carry out evaluations on an ongoing basis using an array with the same specifications.

### (Comparative example)

The procedure and results for an evaluation carried out using an existing method (Non Patent Document 1) are shown below. As in the embodiment, *Enterococcus faecalis* and *Pseudoramibacter alactolyticus* were used as the detection target microorganisms from among the microbial species present in the oral cavity.

### (1) Fabrication of the DNA microarray

Using for the capture probe an aqueous solution prepared by dissolving a synthetic oligo DNA (Nihon Gene Research Laboratories Inc.) modified by the amino group at the 3' terminal, a geneslide (Toyo Kohan Co., Ltd.) glass slide was spotted using a GENESHOT (registered trademark) spotter from NGK Insulators, Ltd. Spotting was followed by baking for 1 hour at 80°C. The immobilization of the synthetic oligo DNAwas carried out by the same procedure as in the embodiment. The following sequences were used in the capture probes for detection of each of the aforementioned microorganisms.
probe for *Enterococcus faecalis* detection: 5-ACCACTCTAGAGATA-3' (SEQ ID NO:108)
probe for *Pseudoramibacter alactolyticus* detection: 5'-AGCGCAATAGAGATA-3' (SEQ ID NO: 109)

### (2) Target nucleic acid amplification and labeling

In order to amplify the target nucleic acids originating with the individual microorganisms, target nucleic acid amplification primers were used that had the same sequence as in the embodiment and that had Cy3 at the 5' terminal.
F-primer: 5-Cy3-AGGTTAAAACTCAAAGGAATTGACG-3'(SEQ ID NO: 101)
R-primer: 5'-Cy3-ATGGTGTGACGGGCGGTGTGT-3' (SEQ ID NO: 102)

PCR was run under the conditions given below on the DNAs extracted from the aforementioned microorganisms to provide comparative examples 1 and 2, respectively. The amplified sample gene was purified with a MinElute PCR Purification Kit from QIAGEN, followed by confirmation that amplification had occurred for the intended length.

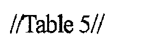

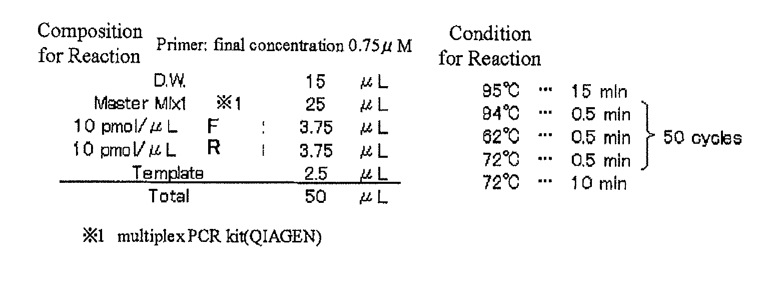

### (3) Hybridization using the DNA microarray

The protocols given below were used for reaction with the DNA microarray using the labeled comparative examples 1 and 2 and for washing. Washing was carried out by transferring the wash solution to a glass staining tank; immersing the glass slide after the completion of the hybridization reaction; and shaking up and down for 5 minutes. The glass slide was additionally transferred into a glass staining tank containing sterile water and up-and-down shaking was carried out for 1 minute. The residual moisture on the surface of the glass slide was then removed by centrifugal drying for 1 minute at 2000 rpm. //Table 6// Target Solution : (PCR Product 10 µ L + Hybridization buffer 40 µL) /reaction Denaturation : Heat Denaturation (95 °C 3 mill → Strage in ice 3 min) Hybridize : 50 °C, 2hr Wash : 2 × SSC , RT 5 min, using shaker

### (4) Detection of fluorescence with a scanner

Using an ArrayWoRx from Applied Precision, a fluorescent image was acquired with adjustment of the photoexposure time as appropriate.

### (5) Data analysis

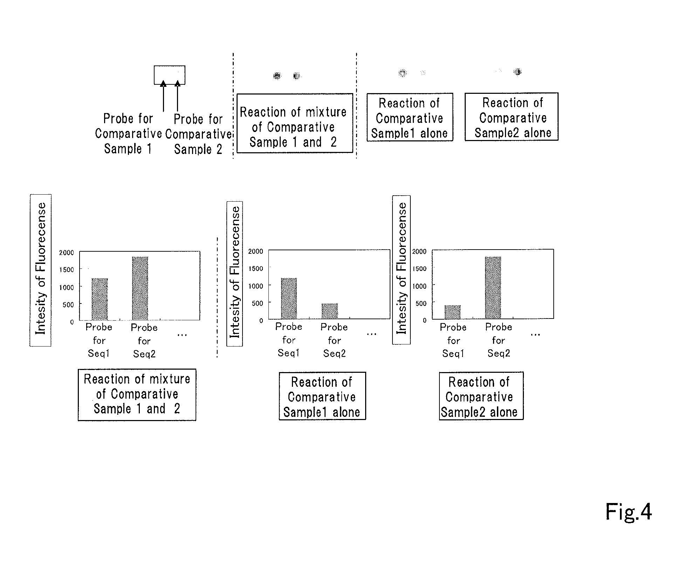

Using GenePix Pro as the image digitization software, digitization was carried out on the fluorescent signal in the obtained image. The results are shown in FIG 4.

As shown in FIG 4, when the hybridization reaction was run using a mixture of comparative samples 1 and 2, a fluorescent signal was yielded by the reaction for both samples, which indicated that sample detection was possible. On the other hand, when the individual hybridization reactions were run without mixing comparative samples 1 and 2, a fluorescent signal (about 10%) was obtained, although weakly, for the nontarget probe, which demonstrated that a nonspecific reaction by the sample had occurred.

The time required for hybridization in this comparative example was about 2 hours. In addition, a nonspecific reaction (about 10% as the fluorescence intensity) by the labeled sample to the nontarget probe (sequence not a complete match) was seen on the DNA microarray. On the other hand, in the case of the embodiment, the time required for hybridization could be shortened to about 30 minutes and the nonspecific reaction by the labeled sample to the nontarget probe (sequence not a complete match) on the DNA microarray could be substantially reduced (less than 1% as the fluorescence intensity).

Thus, according to the present invention, hybridization can be carried out generally at a constant temperature (about 37°C) and in a time of about 30 minutes (one-fourth that of conventional methods) and a nucleic acid can be detected in a particular genome more accurately than heretofore and sequence determination can be carried out more accurately than heretofore.

### Sequence Listing Free Text

SEQ ID NOs 1 to 100, 103, 104, 105, 106, 108, 109: probe
SEQ ID NOs 101, 102,107: primer

## Claims

1. A method of detecting or analyzing a target sequence in a genomic DNA by using a capture probe immobilized on a solid carrier,
the method comprising:
bringing the target nucleic acid into contact with a first query probe that has a sequence complementary to a portion of the target sequence or to a sequence adjacent to the portion and a second query probe that has a sequence complementary to another portion of the target sequence or to a sequence adjacent to the another portion and a recognition sequence complementary to a portion of the capture probe;
acquiring a ligated molecule by ligating the first query probe and the second query probe that are hybridized to the target nucleic acid;
bringing the ligated molecule into contact with the capture probe on the solid carrier and then capturing the ligated molecule on the solid carrier by hybridizing the capture probe with the recognition sequence in the ligated molecule; and
detecting the captured ligated molecule.

2. The method according to claim 1, wherein the contacting step uses a plurality of first query probes and a plurality of second query probes in order to bring these probes into simultaneous contact with a plurality of target nucleic acids.

3. The method according to claim 2, which, based on the plurality of target sequences, detects or identifies a source organism or source organisms for a single type of genomic DNA or for two or more types of genomic DNAs.

4. The method according to any of claims 1 to 3, comprising, prior to the detecting step, labeling any selection from the group consisting of the first query probe, the second query probe, and the ligated molecule, wherein
the detection step is a step of detecting a signal that is based on this labeling.

5. The method according to claim 4, wherein the labeling step labeles the ligated molecule while amplifying ligated molecule.

6. The method according to any of claims 1 to 5, wherein the ligated molecule acquisitiing step ligates the first query probe and the second query probe with a ligase.

7. The method according to any of claims 1 to 6, comprising, prior to the contacting step, amplifying the DNA

8. The method according to any of claims 1 to 7, wherein the genomic DNA originates from any selection from viruses and microorganisms that are targets for diagnosis or testing in case of food sanitation and diseases, and wherein the detecting step detects or identifies the viruses or microorganisms.

9. A microarray used in the method according to any of claims 1 to 8, wherein the capture probe immobilized in the microarray is composed of at least one of capture probes having any base sequence selected from base sequences described in SEQ ID NOs: 1 to 100 and base sequences that are complementary to these base sequences.
